# EUROPEAN PATENT APPLICATION

(11) **EP 2 457 512 A1**
(43) Date of publication of application: **30.05.2012**
(21) Application number: 11189671.8
(22) Date of filing: 18.11.2011
(51) Int. Cl.: A61B 6/00, G06T 7/00, G06F 19/00

(54) **System and method for including and correcting subject orientation data in digital radiographic images**

(30) Priority: 27.11.2010 US 954862
(71) Applicant: General Electric Company, Schenectady, NY 12345 (US)
(72) Inventor: Mabini, Daniel Pol, Waukesha, Wisconsin 53188 (US); Arista, Karla Angelica, Waukesha, Wisconsin 53188 (US); Schulte, Ellyn Mae, Waukesha, Wisconsin 53188 (US)
(74) Representative: Bedford, Grant Richard

(57) **Abstract**

A system and method for including and correcting subject orientation data in a digital radiographic image. A subject (14) is positioned in an X-ray imaging system (10) and an orientation of the subject (14) is entered into the X-ray imaging system (10). The subject is imaged with an X-ray exposure to create an X-ray image (12) of the subject (10). An operator reviews the X-ray image (12) of the subject (12) and subject orientation data. If the subject orientation data is not correct, the operator may correct the subject orientation data so that it matches the subject's anatomy during the X-ray exposure. The correct subject orientation data is then saved and is part of the X-ray image (12) and Digital Imaging and Communications in Medicine (DICOM) header when sent to a Picture Archiving and Communication System (PACS).

## Description

This disclosure relates generally to X-ray imaging systems, and more particularly to a system and method for including and correcting subject orientation data in digital radiographic images.

Radiographic imaging systems, such as X-ray imaging systems, are used to generate images showing internal structures or features of a subject. In modem X-ray imaging systems, X-rays are generated by an X-ray source and are directed towards a patient or other subject. The X-rays travel through the subject, are absorbed or attenuated by internal structures of the subject, and impact a film, an imaging plate (cassette) or a digital detector where image data is generated. The image data is processed and reconstructed into a useful X-ray image. Similar techniques are used for computed tomography, fluoroscopy, and tomosynthesis image generation. In a medical context, for example, such X-ray imaging systems may be used for viewing internal anatomies, tissues and organs of a subject for screening or diagnostic purposes. In other contexts, parts, structures, baggage, parcels, and other subjects may be imaged to assess their contents, structural integrity or other purposes.

X-ray imaging generally includes positioning a subject within an X-ray imaging system, initiating an X-ray exposure by directing generated X-rays towards the subject, and acquiring X-ray images of the subject. Often times during use, a review of the X-ray images may reveal errors related to image quality or positioning of the subject. The X-ray images also typically include orientation data in order to identify the position and orientation of the subject during the X-ray exposure. The orientation data identifies the head, foot, anterior, posterior, right, and left sides of the subject on the processed X-ray image and in the Digital Imaging and Communications in Medicine (DICOM) header. However, this orientation data may be incorrectly identified on the processed X-ray image and in the DICOM header. For example, the head of a subject may be labeled as a foot, or the left side of a subject may be labeled as the right side, and vice versa, etc. Errors in the orientation data may lead to misdiagnosis and catastrophic errors occurring during treatment of medical condition, for example, by treating or operating on the wrong anatomy of a subject.

Therefore, there is a need for an X-ray imaging system and method that allows for revision of the orientation data of a subject on an X-ray image and in the DICOM header, if necessary, to match the subject's anatomy during an X-ray exposure.

In accordance with an aspect of the disclosure there is provided, a method of X-ray imaging, comprising positioning a subject in an X-ray imaging system, imaging the subject with an X-ray exposure to obtain an X-ray image, reviewing the X-ray image and an orientation of the subject with respect to the X-ray image to determine if the orientation of the subject is correct, revising an incorrect orientation of the subject to a correct orientation of the subject, and saving the correct orientation of the subject with the X-ray image.

In accordance with an aspect of the disclosure, a method of X-ray imaging, comprising positioning a subject in an X-ray imaging system, entering an orientation of the subject in the X-ray imaging system, imaging the subject with an X-ray exposure to obtain an X-ray image, reviewing the X-ray image and the orientation of the subject with respect to the X-ray image to determine if the orientation of the subject is correct, revising an incorrect orientation of the subject to a correct orientation of the subject after the X-ray exposure, and saving the correct orientation of the subject with the X-ray image and a DICOM header.

In accordance with an aspect of the disclosure, an X-ray imaging system, comprising an X-ray source, an X-ray detector spaced apart from the X-ray source, a control and processing system coupled to and controlling operation of the X-ray source and the X-ray detector, an operator interface computer coupled to the control and processing system, and a display monitor coupled to the operator interface computer for displaying an X-ray image and a subject orientation data label associated with the X-ray image, wherein the subject orientation data label is selectively added to the X-ray image for verifying a correct orientation of a subject on the X-ray image, and wherein the operator interface computer and the display monitor are used for reviewing and correcting an incorrect subject orientation data label on the X-ray image after an X-ray exposure.

Various other features, aspects, and advantages will be made apparent to those skilled in the art from the accompanying drawings and detailed description thereof. In the drawings:
FIG. 1 is a block diagram of an exemplary embodiment of a digital X-ray imaging system designed to provide orientation data of a subject being imaged on a digital X-ray image;
FIG. 2 is a flow diagram of an exemplary embodiment of a method for reviewing and revising orientation data of a subject being imaged;
FIG. 3 is an exemplary embodiment of an acquisition tool of the digital X-ray imaging system of FIG. 1 for entering or selecting X-ray exposure parameters, subject position and orientation parameters;
FIG. 4 is an exemplary embodiment of a subject orientation data label that may be selectively added to a digital X-ray image to indicate subject orientation; and
FIG. 5 is an exemplary embodiment of the subject orientation data label of FIG. 4 superimposed on a digital X-ray image to indicate subject orientation.

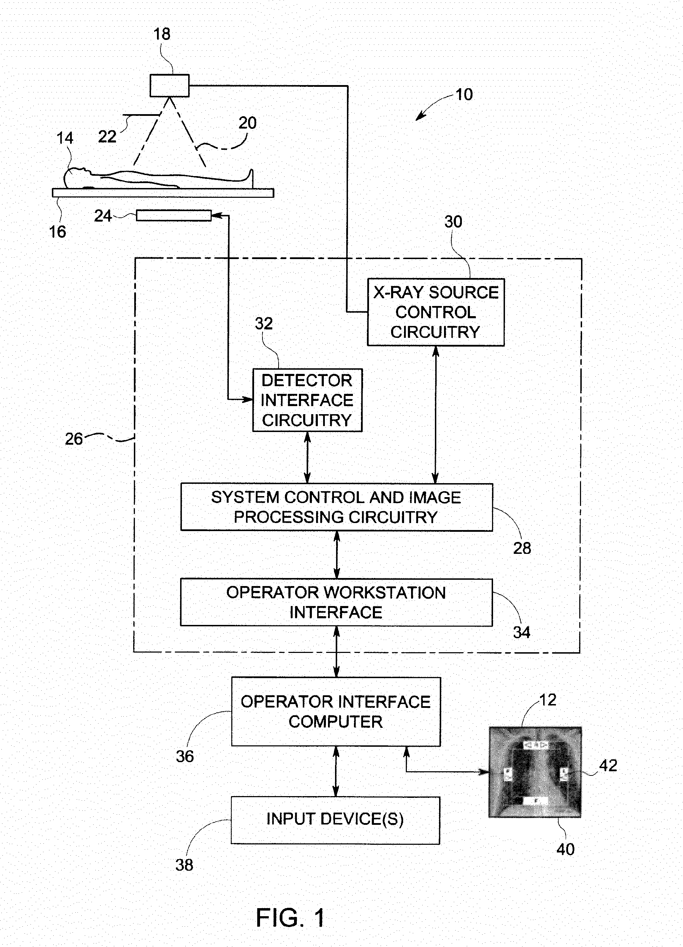

Referring to the drawings, FIG. 1 illustrates a block diagram of an exemplary embodiment of a digital x-ray imaging system 10 designed to provide orientation data of a subject being imaged on a digital X-ray image. It should be appreciated by those skilled in the art that this disclosure is applicable to different types of digital x-ray imaging systems, such as digital radiography (RAD), mammography, tomosynthesis, vascular and computed tomography (CT) imaging systems. The digital X-ray imaging system 10 includes an X-ray source 18, a collimator 22 adjacent to the X-ray source 18, a subject 14 to be imaged, and a digital X-ray detector 24.

The digital X-ray imaging system 10 is designed to create images of the subject 14 by means of an X-ray beam 20 emitted by X-ray source 18, and passing through collimator 22, which forms and confines the X-ray beam 20 to a desired region, wherein the subject 14, such as a human patient, an animal or an object, is positioned. A portion of the X-ray beam 20 passes through or around the subject 14, and is altered by attenuation and/or absorption by tissues within the subject 14, continues on toward and impacts the digital X-ray detector 24. In an exemplary embodiment, the digital X-ray detector 24 may be a fixed detector or a portable detector. In an exemplary embodiment, the digital X-ray detector 24 may be a digital flat panel x-ray detector. The digital X-ray detector 24 converts X-ray photons received on its surface to lower energy light photons, and subsequently to electric signals, which are acquired and processed to reconstruct an image of internal anatomy within the subject 14. In an exemplary embodiment, the digital X-ray imaging system may be a fixed X-ray imaging system, including a support 16 for supporting the subject 14 being imaged, such as a table or a wall stand, or a mobile X-ray imaging system that may be transported to different areas of a facility.

The digital X-ray imaging system 10 further includes a control and processing system 26 coupled to X-ray source 18 and digital X-ray detector 24 for controlling operation of the X-ray source 18 and digital X-ray detector 24. The control and processing system 26 may supply both power and control signals for imaging examination sequences, and collect imaging data for reconstruction of useful images. In general, control and processing system 26 commands operation of the digital X-ray imaging system 10 to execute examination protocols and to process acquired image data. The control and processing system 26 may also include signal processing circuitry, based on a general purpose or application-specific computer, associated memory circuitry for storing programs and routines executed by the computer, as well as configuration parameters and image data, interface circuits, and so forth.

In the illustrated embodiment, for example, the control and processing system 26 includes system control and image processing circuitry 28. Such circuitry typically includes a programmed processor, supporting memory, specific applications executed by the processor during operation, and so forth. The system control and image processing circuitry 28 may be coupled to X-ray source control circuitry 30 that itself allows for control of operation of the X-ray source 18. The X-ray source control circuitry 30 may, for example, under the direction of the system control and image data processing circuitry 28, regulate the current and voltage applied to the X-ray source 18, trigger the generation of X-rays from the X-ray source 18, trigger startup and shutdown sequences of the X-ray source 18, and so forth.

The system control and image data processing circuitry 28 is further coupled to detector interface circuitry 32. The detector interface circuitry 32 allows for enabling the digital X-ray detector 24, and for collecting imaging data from the digital X-ray detector 24. As will be appreciated by those skilled in the art, various designs and operations of such detectors and detector interface circuitry are known and are presently in use. Such designs will typically include detectors having an array of discrete pixel elements defined by solid state switches and photodiodes. The impacting radiation affects the charge of the photodiodes, and the switches allow for collection of information regarding the impacting radiation (e.g., depletion of charge of the photodiodes). The information may then be processed to develop detailed images in which gray levels or other features of individual pixels in an image are indicative of the radiation impacting corresponding regions of the detector.

The control and processing system 26 is also illustrated as including an operator workstation interface 34. The operator workstation interface 34 allows for interaction by an operator who will typically provide inputs through an operator interface computer 36. The operator interface computer 36 and/or the system control and image data processing circuitry 28 may perform filtering functions, control functions, image reconstruction functions, and so forth. One or more input devices 38 may be coupled to the operator interface computer 36, such as a touch screen, keyboard, computer mouse, remote control, and other input devices. The operator interface computer 36 is further coupled to a display monitor 40 on which X-ray images 12 may be displayed, instructions and X-ray acquisition tools may be provided, and so forth. In general, the operator interface computer 36 may include memory and programs sufficient for displaying the desired X-ray images 12, and for performing certain manipulative X-ray imaging system functions. It should be further noted that the operator interface computer 36 may be coupled to a Picture Archiving and Communication System (PACS). Such a PACS might be coupled to remote clients, such as a radiology department information system or hospital information system, or to an internal or external network, so that others at different locations may gain access to image data.

The digital X-ray imaging system 10 illustrated in FIG. 1 is adapted to allow for reviewing and revising orientation data of a subject 14 being imaged on a digital X-ray image 12. In particular, a subject orientation data label 42 may be added to or superimposed on the X-ray image 12 in order to correctly identify the position and orientation of the subject's anatomy during an X-ray exposure. The subject orientation data label 42 may be employed to verify and correct orientation of the subject within the X-ray field of the digital X-ray imaging system. If it is determined that the orientation is not correct, than an operator of the digital X-ray imaging system may correct the subject orientation to match the subject's anatomy during an X-ray exposure. Consequently, this may prevent a misdiagnosis and catastrophic errors occurring during treatment of medical condition, for example, by treating or operating on the wrong anatomy of a subject.

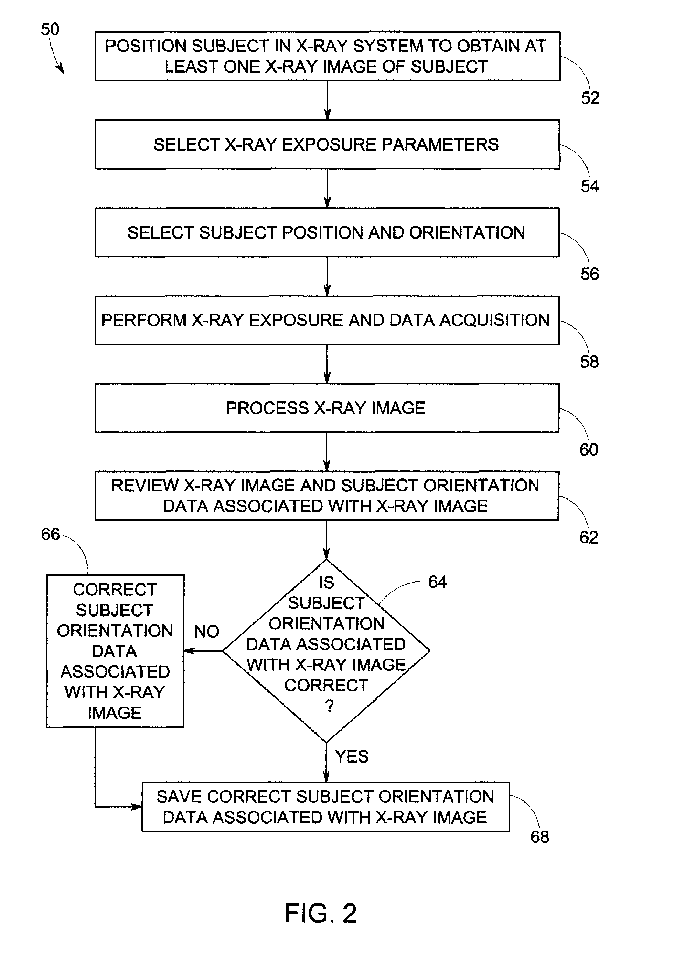

FIG. 2 illustrates a flow diagram of an exemplary embodiment of a method 50 for reviewing and revising orientation data of a subject being imaged. The method 50 is preferably implemented on a digital X-ray imaging system 10. The digital X-ray imaging system 10 may display the orientation data of a subject being imaged, and allows an operator of the digital X-ray imaging system 10 to review and revise the subject orientation data on an X-ray image and in the DICOM header, if necessary, to match the subject's anatomy during an X-ray exposure.

The first step 52 of the method 50 includes positioning a subject within a digital X-ray imaging system in order to obtain at least one X-ray image of the subject. In an exemplary embodiment, the subject may be a human patient, an animal or an object.

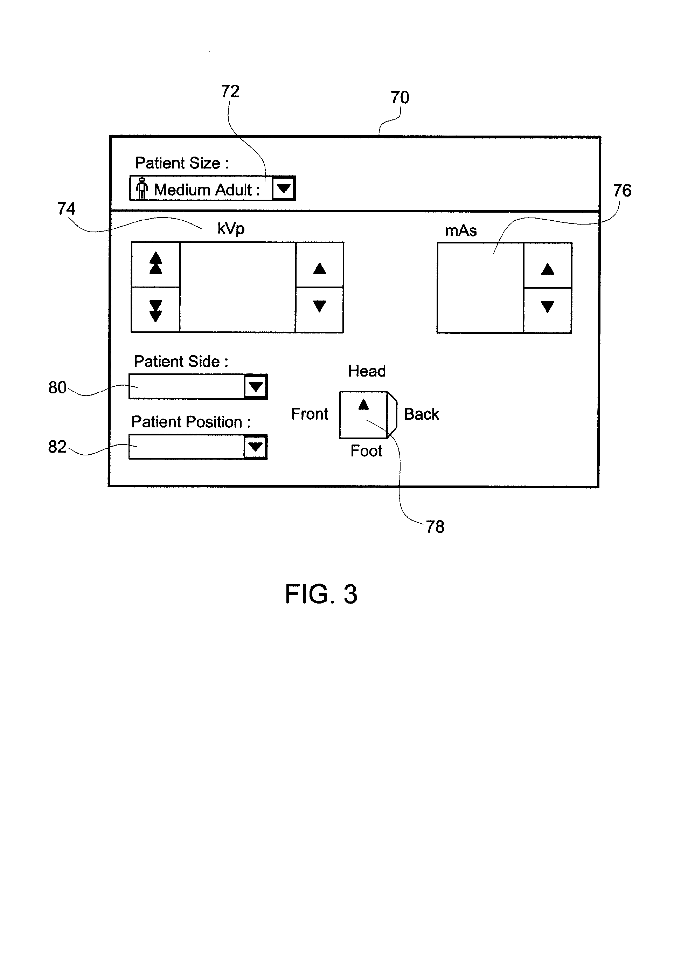

The next steps 54 and 56 of method 50 include an operator entering or selecting X-ray exposure parameters, and subject position and orientation parameters using an X-ray acquisition tool 70 on the operator interface computer 36. FIG. 3 illustrates an exemplary embodiment of an X-ray acquisition tool 70 of the digital X-ray imaging system 10 of FIG. 1 for entering or selecting X-ray exposure parameters, and subject position and orientation parameters. In an exemplary embodiment, the X-ray acquisition tool 70 may include X-ray exposure parameters, such as a patient size parameter 72 for entering or selecting the appropriate size of the subject being imaged; and a kVp parameter and a mAs parameter for entering or selecting the desired peak kilovoltage and milliampere-seconds of the X-ray source. The X-ray acquisition tool 70 may also include subject position and orientation parameters, such as a patient side parameter and a patient position parameter for entering or selecting the appropriate side (e.g., left side, right side) and position (e.g, head-up position, head-down position) of the subject being imaged. The X-ray acquisition tool 70 may also include detector symbol 78 with an arrow associating the left side of the detector with the head of the subject being imaged.

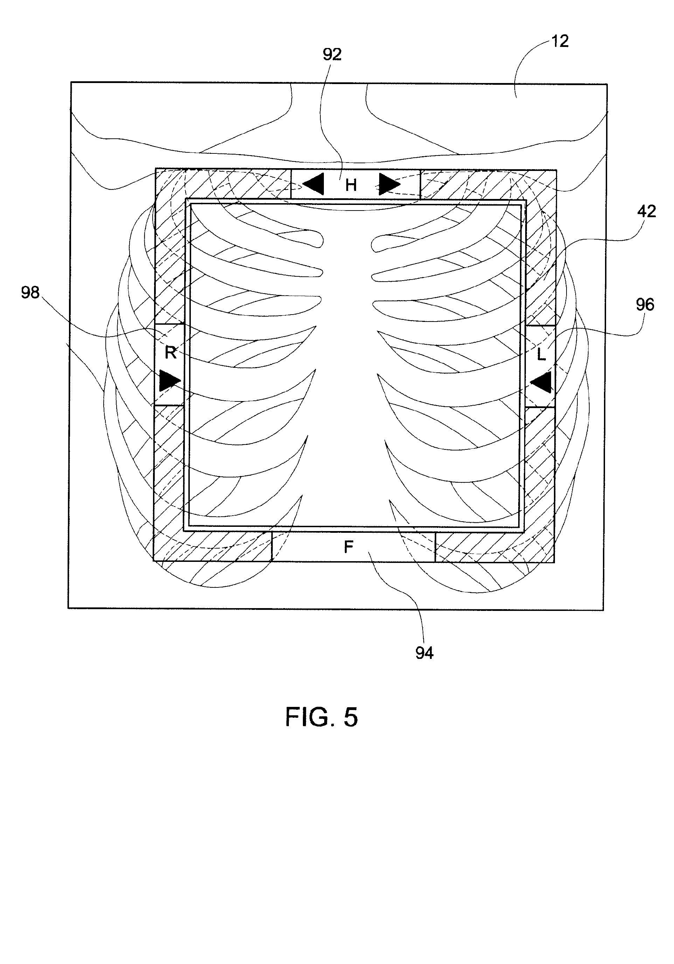

Step 58 of method 50 includes the operator initiating an X-ray exposure and image data acquisition. Step 60 of method 50 includes processing and reconstructing the image data to create a digital X-ray image that may be visually displayed on display monitor 40 of the digital X-ray imaging system. Step 62 of method 50 includes the operator reviewing the digital X-ray image on the display monitor 40. The digital X-ray image may be displayed with a subject orientation data label 42 as shown in FIG. 5.

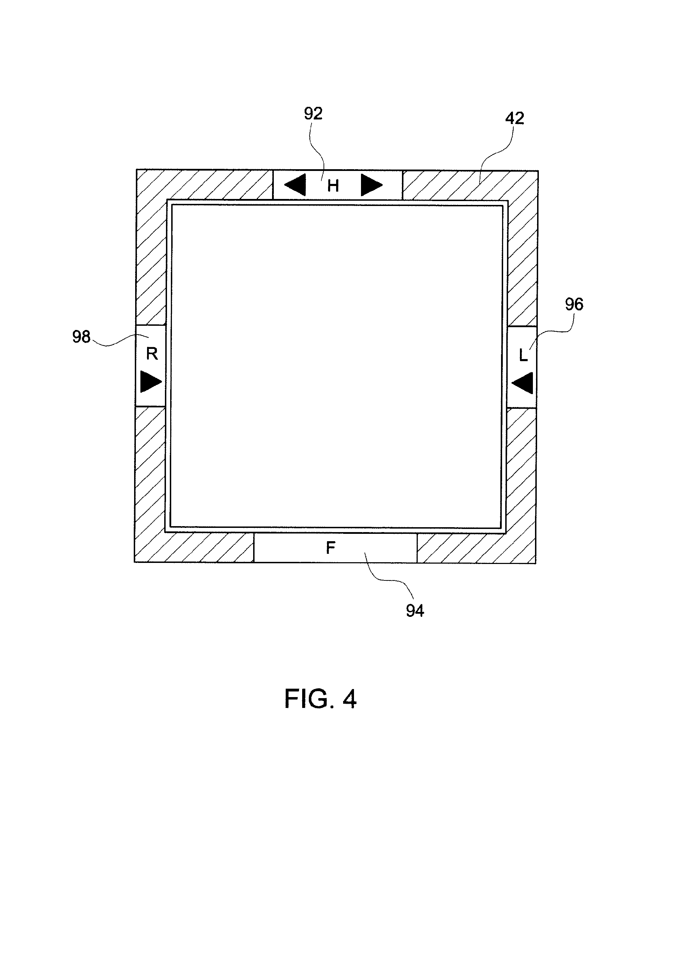

FIG. 4 illustrates an exemplary embodiment of a subject orientation data label 42 that may be selectively added to the digital X-ray image to indicate subject orientation. The subject orientation data label 42 may include a plurality of symbols representing the subject anatomical orientation. For example, the plurality of symbols may be designated by capital letters, such as an H label 92 representing the anatomical head of the subject being imaged, an F label 94 representing the anatomical foot of the subject being imaged, an L label 96 representing the anatomical left side of the subject being imaged, and an R label 98 representing the anatomical right side of the subject being imaged. The plurality of symbols may further include an A label representing the anatomical anterior of the subject being imaged and a P label representing the anatomical posterior of the subject being imaged.

FIG. 5 illustrates an exemplary embodiment of the subject orientation data label 42 of FIG. 4 added or superimposed on a digital X-ray image 12 to indicate the subject orientation on the X-ray image. The subject orientation data label 42 represents the orientation of the subject anatomically. In other words, the subject orientation data label 42 is a conceptual example of what an operator might see in order to determine whether or not to revise the subject orientation on the X-ray image and DICOM header.

Returning to step 62, the operator reviews the X-ray image and the subject orientation data associated with the X-ray image in order to determine whether the subject orientation data associated with the X-ray image is correct or not. This decision is determined at step 64 of method 50. The operator has option to correct the subject orientation data associated with the X-ray image if it is not correct. This action may be performed in an acquisition mode or a review mode of the image acquisition workflow. During the acquisition mode, the subject orientation data is corrected on the original X-ray image. During the review mode, a new X-ray image is generated with corrected subject orientation data. Step 64 provides the operator with the ability to correct subject orientation data on the X-ray image and DICOM header after an X-ray exposure. The decision to correct the subject orientation is made by the operator visualizing the displayed X-ray image and the subject orientation data. That is, the operator observes the X-ray image and the subject orientation data, and makes a decision whether or not to correct the subject orientation data, if necessary.

At step 66, if the subject orientation data is determined by the operator to not be correct, then the operator corrects the subject orientation data to match the anatomy of the subject. Finally, at step 68, if the subject orientation data is determined by the operator to be correct, then the correct subject orientation data is saved and associated with the X-ray image and the DICOM header. The saved correct subject orientation data is part of the image and DICOM header when sent to PACS.

While the disclosure has been described with reference to various embodiments, those skilled in the art will appreciate that certain substitutions, alterations and omissions may be made to the embodiments without departing from the spirit of the disclosure.

Accordingly, the foregoing description is meant to be exemplary only, and should not limit the scope of the disclosure as set forth in the following claims.

Various aspects and embodiments of the present invention are defined by the following numbered clauses:
1. A method of X-ray imaging, comprising:
   positioning a subject in an X-ray imaging system; imaging the subject with an X-ray exposure to obtain an X-ray image;
   reviewing the X-ray image and an orientation of the subject with respect to the X-ray image to determine if the orientation of the subject is correct;
   revising an incorrect orientation of the subject to a correct orientation of the subject; and
   saving the correct orientation of the subject with the X-ray image.
2. The method of clause 1, wherein the reviewing step includes comparing subject orientation data with the X-ray image.
3. The method of any preceding clause, wherein the reviewing step includes adding a subject orientation data label to the X-ray image and comparing a subject orientation data label with the X-ray image.
4. The method of any preceding clause, wherein the subject orientation data label includes symbols representing subject anatomical orientation.
5. The method of any preceding clause, wherein the symbols include H for head, F for foot, L for left, R for right, A for anterior, and P for posterior.
6. The method of any preceding clause, wherein the saving step includes associating the correct orientation of the subject with the X-ray image and a Digital Imaging and Communications in Medicine (DICOM) header.
7. The method of any preceding clause, wherein the correct orientation of the subject is part of the X-ray image and a DICOM header when sent to a Picture Archiving and Communication System (PACS).
8. The method of any preceding clause, wherein the X-ray image is a digital X-ray image.
9. A method of X-ray imaging, comprising:
   positioning a subject in an X-ray imaging system;
   entering an orientation of the subject in the X-ray imaging system;
   imaging the subject with an X-ray exposure to obtain an X-ray image;
   reviewing the X-ray image and the orientation of the subject with respect to the X-ray image to determine if the orientation of the subject is correct;
   revising an incorrect orientation of the subject to a correct orientation of the subject after the X-ray exposure; and
   saving the correct orientation of the subject with the X-ray image and a DICOM header.
10. The method of any preceding clause, wherein the reviewing step includes comparing subject orientation data with the X-ray image.
11. The method of any preceding clause, wherein the reviewing step includes superimposing a subject orientation data label on the X-ray image and comparing a subject orientation data label with the X-ray image.
12. The method of any preceding clause, wherein the subject orientation data label includes symbols representing subject anatomical orientation.
13. The method of any preceding clause, wherein the symbols include H for head, F for foot, L for left, R for right, A for anterior, and P for posterior.
14. The method of any preceding clause, wherein the saving step includes associating the correct orientation of the subject with the X-ray image and a DICOM header.
15. The method of any preceding clause, wherein the correct orientation of the subject is part of the X-ray image and a DICOM header when sent to a PACS.
16. The method of any preceding clause, wherein the X-ray image is a digital X-ray image.
17. An X-ray imaging system, comprising:
   an X-ray source;
   an X-ray detector spaced apart from the X-ray source;
   a control and processing system coupled to and controlling operation of the X-ray source and the X-ray detector;
   an operator interface computer coupled to the control and processing system; and
   a display monitor coupled to the operator interface computer for displaying an X-ray image and a subject orientation data label associated with the X-ray image;
   wherein the subject orientation data label is selectively added to the X-ray image for verifying a correct orientation of a subject on the X-ray image; and
   wherein the operator interface computer and the display monitor are used for reviewing and correcting an incorrect subject orientation data label on the X-ray image after an X-ray exposure.
18. The system of any preceding clause, wherein the subject orientation data label includes symbols representing subject anatomical orientation.
19. The system of any preceding clause, wherein the symbols include H for head, F for foot, L for left, R for right, A for anterior, and P for posterior.
20. The system of any preceding clause, wherein the X-ray image is a digital X-ray image.

## Claims

1. A method (50) of X-ray imaging, comprising:
positioning a subject in an X-ray imaging system (52);
imaging the subject with an X-ray exposure to obtain an X-ray image (58);
reviewing the X-ray image and an orientation of the subject with respect to the X-ray image to determine if the orientation of the subject is correct (62);
revising an incorrect orientation of the subject to a correct orientation of the subject (66); and
saving the correct orientation of the subject with the X-ray image (68).

2. The method (50) of claim 1, wherein the reviewing step (62) includes comparing subject orientation data with the X-ray image (12).

3. The method (50) of any preceding claim, wherein the reviewing step (62) includes adding a subject orientation data label (42) to the X-ray image (12) and comparing the subject orientation data label (42) with the X-ray image (12).

4. The method (50) of claim 3, wherein the subject orientation data label (42) includes symbols (92, 94, 96, 98) representing subject anatomical orientation.

5. The method (50) of claim 4, wherein the symbols (92, 94, 96, 98) include H for head, F for foot, L for left, R for right, A for anterior, and P for posterior.

6. The method (50) of any preceding claim, wherein the saving step (68) includes associating the correct orientation of the subject (14) with the X-ray image (12) and a Digital Imaging and Communications in Medicine (DICOM) header.

7. The method (50) of any preceding claim, wherein the correct orientation of the subject (14) is part of the X-ray image (12) and a DICOM header when sent to a Picture Archiving and Communication System (PACS).

8. The method (50) of any preceding claim, wherein the X-ray image (12) is a digital X-ray image.

9. An X-ray imaging system (10), comprising:
an X-ray source (18);
an X-ray detector (24) spaced apart from the X-ray source (18);
a control and processing system (26) coupled to and controlling operation of the X-ray source (18) and the X-ray detector (24);
an operator interface computer (36) coupled to the control and processing system (26); and
a display monitor (40) coupled to the operator interface computer (36) for displaying an X-ray image (12) and a subject orientation data label (42) associated with the X-ray image (12);
wherein the subject orientation data label (42) is selectively added to the X-ray image (12) for verifying a correct orientation of a subject (14) on the X-ray image (12); and
wherein the operator interface computer (36) and the display monitor (40) are used for reviewing and correcting an incorrect subject orientation data label on the X-ray image (12) after an X-ray exposure.

10. The system (10) of claim 9, wherein the subject orientation data label (42) includes symbols (92, 94, 96, 98) representing subject anatomical orientation.
